# EUROPEAN PATENT SPECIFICATION

(11) **EP 3 123 963 B1**
(45) Date of publication and mention of the grant of the patent: **30.10.2019**
(21) Application number: 15769782.2
(22) Date of filing: 26.03.2015
(51) Int. Cl.: A61B 17/29, A61B 17/34, A61B 90/50, A61B 1/05, A61B 1/00

(54) **ENDOSCOPE SURGICAL DEVICE, SURGICAL TOOL AND GUIDE MEMBER**
CHIRURGISCHE ENDOSKOPVORRICHTUNG, CHIRURGISCHES WERKZEUG UND FÜHRUNGSELEMENT
DISPOSITIF CHIRURGICAL D'ENDOSCOPE, OUTIL CHIRURGICAL ET ÉLÉMENT DE GUIDAGE

(30) Priority: 27.03.2014 US 201461971258 P
(43) Date of publication of application: 01.02.2017
(73) Proprietor: Fujifilm Corporation, Minato-ku Tokyo 106-8620 (JP)
(72) Inventor: IWASAKA, Masayuki, Ashigarakami-gun Kanagawa 258-8538 (JP); YAMAKAWA, Shinichi, Ashigarakami-gun Kanagawa 258-8538 (JP)
(74) Representative: Klunker IP Patentanwälte PartG mbB
(86) International application number: PCT/JP2015/059354
(87) International publication number: WO 2015/147158

(56) References cited:
- EP-A2- 2 163 217
- EP-A2- 2 238 926
- WO-A1-02/089722
- WO-A1-2013/176167
- WO-A1-2013/176167
- WO-A2-98/48688
- DE-A1- 4 429 812
- JP-A- 2004 141 486
- JP-A- 2009 011 495
- US-A- 5 334 187
- US-A1- 2005 004 595
- US-B1- 6 221 007

## Description

### BACKGROUND OF THE INVENTION

### 1. Field of the Invention

The present invention relates to a treatment tool according to the preamble of claim 1, and an endoscopic surgical device.

### 2. Description of the Related Art

In recent years, since invasion to a patient is small compared to surgery in which a laparotomy, a thoracotomy, or the like, is performed, endoscopic surgery using endoscopes (hard endoscopes), such as a laparoscope, has been widely performed. In endoscopic surgery, a plurality of holes are made in a patient's body wall, an endoscope is inserted into a body cavity from one hole of these, and a treatment tool is inserted into the body cavity from another hole. Then, treatment of a living body tissue is performed with the treatment tool while observing the living body tissue within the body cavity with the endoscope.

Generally, in endoscopic surgery, one or a plurality of treatment tools are used simultaneously with the endoscope. Therefore, since it is difficult for one surgeon to simultaneously operate the endoscope and the plurality of treatment tools, for example, a task, such as operating a treatment tool that the surgeon holds with his/her hands while making an assistant called an endoscopic technician operate the endoscope is normally performed.

In this way, in endoscopic surgery, it is usual that the surgeon's hands are occupied by the operation of the treatment tools, and the operation of the endoscope is performed by the assistant. Therefore, in a case where the observation position of the endoscope is changed, the surgeon needs to give sequential instructions to the assistant. Hence, the task of correctly directing the orientation of the endoscope to a direction desired by the surgeon is difficult, and stress is likely to be imposed on the surgeon. Additionally, since the assistant performs an operation after the surgeon issues an instruction, there is a tendency that surgery time is likely to be prolonged. Additionally, the assistant needs to operate the endoscope so as not to interfere with a surgeon's procedure, and the operation is likely to become complicated.

In contrast, the present applicant suggests the following technique. In this technique, an overtube that guides an insertion part of an endoscope and an insertion part of a treatment tool into a body cavity is provided with a tubular overtube body that is inserted in a state where the insertion part of the endoscope and the insertion part of the treatment tool are made to be parallel to each other, a movable body that is movable in an axial direction and has an endoscope holding part and a treatment tool holding part is provided inside the overtube body, the insertion part of the endoscope and the insertion part of the treatment tool are held by the respective holding parts in a state where the insertion parts are made to be parallel to each other, and if the insertion part of the treatment tool is moved in the axial direction, the insertion part of the endoscope also moves in the axial direction in an interlocking manner with this movement (refer to WO2013/176167A). According to this technique, the number of holes made in the patient's body wall can be reduced, the invasion to a patient can be reduced, and the visual field of the endoscope can be easily changed while the surgeon operates the treatment tool without asking for an assistant's help.

Meanwhile, JP1995-275253A (JP-H07-275253A) discloses a technique in which an insertion passage (communication passage) for allowing an insertion part of an endoscope to be inserted therethrough is provided inside the insertion part (shaft part) of a treatment tool, and the insertion part of the endoscope is adapted to be capable of being introduced into a body cavity via this insertion passage. According to this technique, since an observation function other than the original functions of the treatment tool can be used together, it is possible to observe the inside of a body cavity without extracting the treatment tool from an overtube, and shortening of surgery time can be achieved. Additionally, since it is not necessary to increase the number of overtubes, invasion to a patient can be reduced.

Additionally, JP2000-107192A discloses a technique in which an insertion part of an endoscope is attached to an insertion part (rod) of a treatment tool therealong, and both the insertion parts are integrated. According to this technique, since an observation window and an illumination window can be arranged at a distal end of the insertion part of the endoscope, and an actuating part (treatment part) of a distal end of the treatment tool can be arranged at a desired position, one surgeon can easily perform surgery by gripping and operating the endoscope and the treatment tool. In accordance with the preamble of claim 1, JP 2004 141486 A discloses a treatment tool adapted for use of an endoscope surgical device including an endoscope, wherein a cranked part of the endoscope is held by a linked member and is fixed to the link member by a locked screw. The squint angle of the endoscope in relation to a distal end of a treatment tool may be changed. However, the operating range of a treatment tool is restricted so as to remain within the field of view of the endoscope.

DE 44 29 812 A1 discloses an optical pipe guided by a guiding pipe which carries a slider. The slider carries a cutting electrode having a knife.

Further, US 5 334 187 A discloses a catheter system having a guide wire which is moved by means of a slide handle.

### SUMMARY OF THE INVENTION

Meanwhile, further improvement of operability is required in the technique disclosed in WO2013/176167A. For example, it is convenient if change to a visual field suitable for detailed observation and treatment can be performed with the position of the treatment tool being maintained when treatment of a living body tissue is performed. In this case, it is desirable that the operation of changing the visual field of the endoscope without both the surgeon's hands being released from the treatment tool during treatment can be performed.

In contrast, the techniques disclosed in JP1995-275253A (JP-H07-275253A) and JP2000-107192A have a configuration in which the endoscope can be moved forward and backward in an interlocking manner with the forward and backward movement of the endoscope by fixing the endoscope to the treatment tool. However, in order to perform change to a visual field suitable for detailed observation and treatment, a surgeon needs to release his/her hands from the treatment tool to adjust the position of the endoscope or an assistant's hand needs to be used temporarily. There is a problem that this causes a decrease in surgical efficiency.

The invention has been made in view of such circumstances, and an object thereof is to provide a treatment tool, and an endoscopic surgical device that can perform change to a visual field suitable for detailed observation and treatment with a simple operation, and improve surgical efficiency.

In order to achieve the above object, a treatment tool related to an aspect of the invention is defined by claim 1.

According to this aspect, the operating part of the treatment tool is provided with the guide member that guides the cable so as to be movable forward and backward in the axial direction so that the cable is kept from being separated from the surface of the operating part of the treatment tool beyond a fixed distance. Thus, a surgeon can perform the forward and backward movement operation of the endoscope with a finger of a hand operating the treatment tool without releasing his/her hand from the treatment tool. Accordingly, it is possible to perform change to a visual field suitable for detailed observation and treatment with a simple operation, and surgical efficiency can be improved.

In the treatment tool related to the aspect of the invention, an aspect in which the guide member is provided at a position where the cable is capable of being held down by an index finger of a hand operating the operating part is preferable.

In the treatment tool related to the aspect of the invention, an aspect in which the operating part includes a handle part that is gripped by an operator and allows the operator to operate the treatment tool, and in a plan view in which the operating part is viewed from a side opposite to a side where the treatment tool insertion part is formed in an axial direction of the treatment tool insertion part, the guide member is arranged on any of surfaces on an upper side, a lower side, a left side, and a right side of the operating part in a state where the handle part is turned down is preferable. In this aspect, an aspect in which the guide member is arranged on a surface on a right side or a left side of the operating part is preferable.

In the endoscopic surgical device related to a further aspect of the invention an aspect in which an interlocking member including an endoscope-coupled part coupled to the endoscope insertion part inserted through the endoscope insertion passage and a treatment tool-coupled part coupled to the treatment tool insertion part inserted through the treatment tool insertion passage, the interlocking member being arranged inside the overtube body so as to be movable forward and backward is further included is preferable. Here, the interlocking member includes a dead zone where the forward and backward movement of either the endoscope or the treatment tool does not interlock with the movement of the other and a sensing zone where the forward and backward movement of either the endoscope or the treatment tool interlocks with the movement of the other.

According to the invention, a surgeon can perform the forward and backward movement operation of the endoscope with a finger of a hand operating the treatment tool without releasing his/her both hands from the treatment tool. Accordingly, it is possible to perform change to a visual field suitable for detailed observation and treatment with a simple operation, and surgical efficiency can be improved.

### BRIEF DESCRIPTION OF THE DRAWINGS

Fig. 1 is a schematic block diagram of an endoscopic surgical device related to the invention.
Fig. 2 is a plan view illustrating a distal end surface of an endoscope insertion part.
Fig. 3 is an external perspective view illustrating an overtube.
Fig. 4 is a sectional view illustrating the internal structure of the overtube.
Fig. 5 is an enlarged sectional view illustrating a portion of Fig. 4 in an enlarged manner.
Fig. 6 is a sectional view when viewed from arrow 6-6 in Fig. 5.
Fig. 7 is an explanatory view used for the description of the action of the slider.
Fig. 8 is an explanatory view used for the description of the action of the slider.
Fig. 9 is an explanatory view used for the description of the action of the slider.
Fig. 10 is an external view illustrating an operating part of a treatment tool from a right side.
Fig. 11 is an external view illustrating the operating part of the treatment tool from a rear side.
Fig. 12 is a view illustrating an aspect in which the operating part of the treatment tool is gripped, from the left side.
Fig. 13 is an external view illustrating an aspect in which an endoscope is sandwiched between an index finger of a right hand gripping the operating part of the treatment tool, and the operating part, from a right side.
Figs. 14A to 14C are views illustrating an aspect of the operation when only the treatment tool moves forward and backward.
Figs. 15A to 15C are views illustrating an aspect of the operation when only the endoscope moves forward and backward.
Figs. 16A to 16C are views illustrating an aspect of the operation when the endoscope moves forward and backward in an interlocking manner with the treatment tool through an interlocking function of a slider.
Figs. 17A to 17C are views illustrating an aspect of the operation when the endoscope moves forward and backward in an interlocking manner with the treatment tool without resort to the interlocking function of the slider.
Fig. 18 is a view illustrating another embodiment for an installation place of a guide member in the operating part.
Fig. 19 is a view illustrating another embodiment of the guide member in the operating part.

### DESCRIPTION OF THE PREFERRED EMBODIMENTS

Preferred embodiments of the invention will be described below in detail according to the accompanying drawings. In addition, any of the drawings may illustrate main parts in an exaggerated manner for description, and may have dimensions different from actual dimensions.

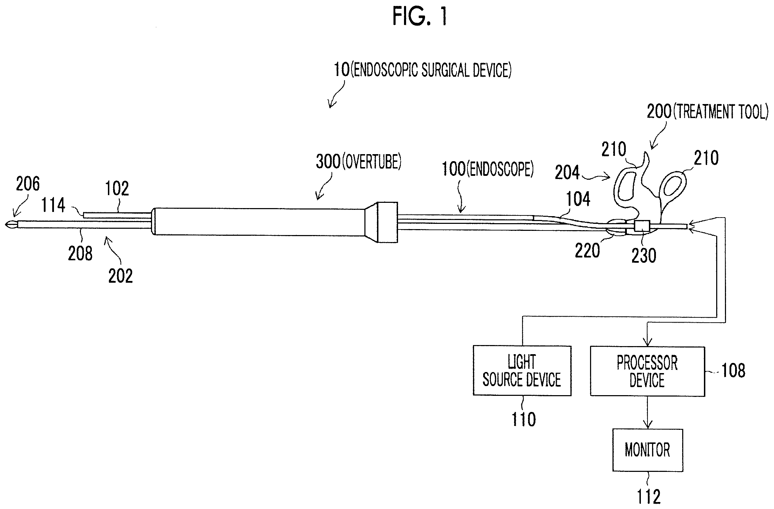

Fig. 1 is a schematic block diagram of an endoscopic surgical device related to the invention. As illustrated in Fig. 1, an endoscopic surgical device 10 includes an endoscope 100 that observes the inside of a patient's body cavity, a treatment tool 200 for examining or treating a diseased site within the patient's body cavity, and an overtube 300 that is inserted into a body wall and guides the endoscope 100 and the treatment tool 200 into the body cavity.

The endoscope 100 is, for example, a hard endoscope, such as a laparoscope, and includes an insertion part 102 (hereinafter referred to as "endoscope insertion part") that is inserted into a body cavity and has an outer peripheral part surrounded by an elongated hard tubular body, and a cable part 104 that is provided continuously with a base end side of the endoscope insertion part 102 and that has an outer peripheral part surrounded by an elongated flexible tubular body.

The cable part 104 indicates a flexible cable portion in which a wire rod, such as a cable or a light guide, which extends from a base end of the endoscope insertion part 102, is housed by covering the wire rod with, for example, a flexible insulating member, such as polyvinyl chloride.

A connector (not illustrated) is provided at an end of the cable part 104 on its extension destination, and each of a processor device 108 and a light source device 110 is detachably connected to the cable part via the connector. Additionally, the processor device 108 is connected to a monitor 112 via a cable.

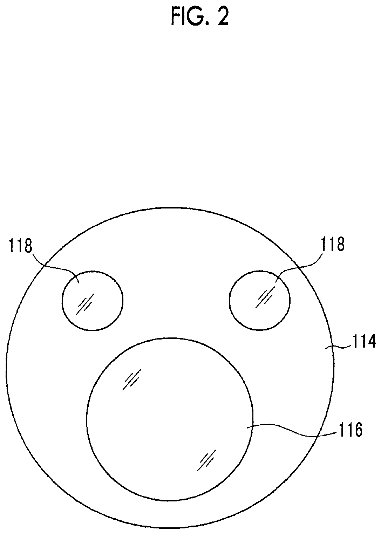

As illustrated in Fig. 2, a distal end surface 114 of the endoscope insertion part 102 is provided with an observation window 116 and illumination windows 118 and 118.

The observation window 116 is a constituent element of an observation part of the endoscope 100, and an objective lens of an observation optical system, and an image pick-up element, such as a charge coupled device (CCD) or a complementary metal oxide semiconductor (CMOS), which is arranged at an image pick-up position of the objective lens, are disposed behind the observation window 116. A signal cape (not illustrated) connected to a substrate that supports this image pickup element is inserted through the endoscope insertion part 102 and the cable part 104 of Fig. 1, is provided to extend up to the connector (not illustrated), and is connected to the processor device 108. An observation image picked up from the observation window 116 is formed on a light-receiving surface of the image pick-up element, and is converted into electrical signals (image pick-up signals), and the electrical signals are output to the processor device 108 via the signal cable and are converted into video signals. Then, the video signals are output to the monitor 112 connected to the processor device 108, and the observation image (endoscope image) is displayed on a screen of the monitor 112.

An exit end of the light guide (not illustrated) is disposed behind the illumination windows 118 and 118 of Fig. 2. The light guide is inserted through the endoscope insertion part 102 and the cable part 104 of Fig. 1 and has an incident end disposed within the connector (not illustrated). Therefore, by coupling the connector to the light source device 110, the illumination light radiated from the light source device 110 is transmitted to the illumination windows 118 and 118 via the light guide, and is radiated forward from the illumination windows 118 and 118. In addition, in Fig. 2, the two illumination windows 118 and 118 are disposed on the distal end surface 114 of the endoscope insertion part 102. However, the number of illumination windows 118 is not limited, and the number thereof may be one or may be three or more. Additionally, the endoscope 100 may not include the light guide.

As illustrated in Fig. 1, the treatment tool 200 consists of, for example, forceps, and includes an elongated insertion part 202 (hereinafter referred to as a "treatment tool insertion part") that is inserted into a body cavity, an operating part 204 that is provided on the base end side of the treatment tool insertion part 202 and is gripped by a surgeon (operator), and a treatment part 206 that is provided on a distal end side of the treatment tool insertion part 202 and is operable by the operation of the operating part 204.

The treatment tool insertion part 202 is provided with a tubular sheath 208, and an operating shaft (not illustrated) that is inserted into the sheath 208 so as to be movable in the direction of an axial center. Moreover, the operating part 204 is provided with a fixed handle 210, and a movable handle 214 that is turnably coupled to the fixed handle 210 via a turning pin. A base end of the operating shaft is coupled to the movable handle 214.

The treatment part 206 is provided with a pair of gripping members that is openable and closable. The gripping members are coupled to a distal end of the operating shaft via a driving mechanism (not illustrated). With the turning operation of the movable handle 214 of the operating part 204, the gripping members of the treatment part 206 are opened and closed via the operating shaft and the driving mechanism.

Additionally, a rotating handle 220 is provided at a base end of the treatment tool insertion part 202 and a distal end of the operating part 204 so as to be turnable around the axis of the treatment tool insertion part 202 (sheath 208). If the rotating handle 220 is rotated, the operating shaft rotates around the axis of the sheath 208, and the pair of gripping members of the treatment part 206 rotates around the axis of the sheath 208 in its entirety via the operating shaft and the driving mechanism.

Moreover, the fixed handle 210 of the operating part 204 is provided with a guide member 230 that guides the cable part 104 so as to be movable forward and backward in the axial direction so that the cable part 104 of the endoscope 100 is kept from being separated from the surface of the operating part 204 beyond a fixed distance. This will be described below.

In addition, the treatment tool 200 is not limited to the forceps, and may be, for example, other treatment tools, such as a laser probe, a suture device, an electric scalpel, a needle holder, and an ultrasonic aspirator.

As illustrated in Fig. 1, the overtube 300 allows the endoscope insertion part 102 and the treatment tool insertion part 202, which are inserted thereinto from the base end side, to be inserted therethrough and delivered from the distal end side. By inserting the overtube 300 into a body wall and having a distal end side thereof arranged outside of the body and a base end side thereof arranged within the body cavity, it is possible to guide the endoscope insertion part 102 and the treatment tool insertion part 202 into the body cavity with one overtube 300. Additionally, the overtube 300 includes an interlocking function of interlocking the endoscope insertion part 102 with the treatment tool insertion part 202 to move these insertion parts forward and backward as will be described below in detail. For example, the endoscope insertion part 102 can also be moved forward and backward by the forward and backward movement operation of only the treatment tool insertion part 202, and a suitable observation image can be obtained without performing the forward and backward movement operation of the endoscope insertion part 102.

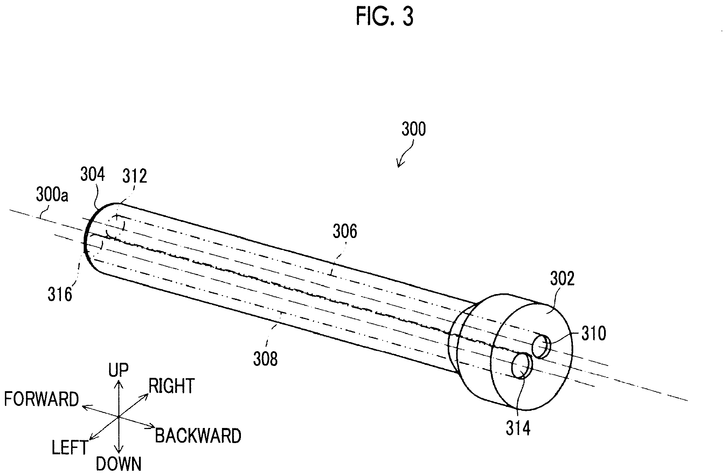

Fig. 3 is an external perspective view illustrating the overtube 300.

As illustrated in this drawing, the overtube 300 has an elongated columnar shape as a whole, and has an endoscope insertion passage 306 through which the endoscope insertion part 102 of the endoscope 100 is inserted so as to be movable forward and backward, and a treatment tool insertion passage 308 through which the treatment tool insertion part 202 of the treatment tool 200 is inserted so as to be movable forward and backward. These insertion passages are parallel to a reference axis 300a (longitudinal axis) indicating a central axis of the overtube.

In addition, regarding the position and orientation of a space where the overtube 300 has been arranged, terms called forward, backward, left, right, up, and down are used with the orientation from the base end surface 302 in a direction along the reference axis 300a to the distal end surface 304 defined as the forward and with the orientation from the reference axis 300a to the endoscope insertion passage 306 defined as the right.

The base end surface 302 of the overtube 300 is provided with an endoscope insertion port 310 that allows the endoscope insertion part 102 to be inserted into the endoscope insertion passage 306 therethrough, and a treatment tool insertion port 314 that allows the treatment tool insertion part 202 to be inserted into the treatment tool insertion passage 308 therethrough.

The distal end surface 304 of the overtube 300 is provided with an endoscope delivery port 312 that allows the endoscope insertion part 102 inserted into the endoscope insertion passage 306 to be delivered to the outside therethrough, and a treatment tool delivery port 316 that allows the treatment tool insertion part 202 inserted into the treatment tool insertion passage 308 to be delivered to the outside therethrough.

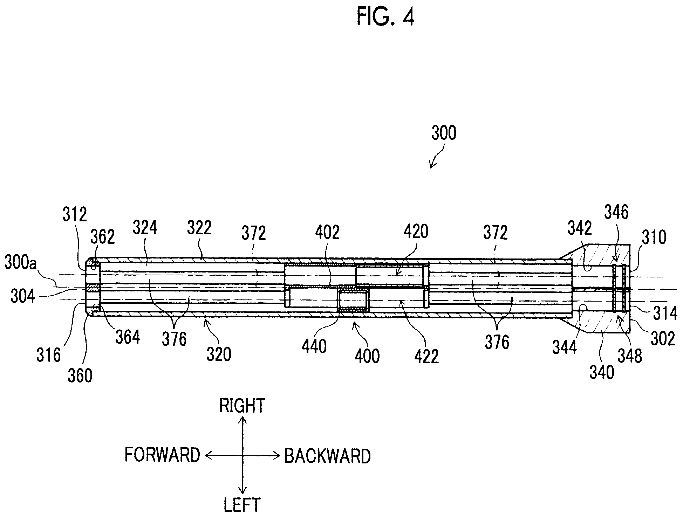

Fig. 4 is a sectional view illustrating the internal structure of the overtube 300, and illustrates a section cut in a plane that includes the reference axis 300a and is orthogonal to an upward-downward direction.

As illustrated in this drawing, the overtube 300 has an overtube body 320 that occupies substantially the entire area in the forward-backward direction, a base end cap 340 that is attached to a rear end (base end) of the overtube 300, a distal end cap 360 that is attached to a distal end, and a slider 400 (the slider 400 is one form of an interlocking member) that is arranged inside the overtube 300.

The overtube body 320 is formed in an elongated cylindrical shape having the reference axis 300a as a central axis using hard resins, metals, or the like, and has an outer wall 322 that surrounds an outer periphery, and a cavity part 324 that penetrates from a base end of the overtube body 320 to a distal end thereof.

The cavity part 324 includes spaces serving as the endoscope insertion passage 306 and the treatment tool insertion passage 308, and houses the slider 400 and the like.

The base end cap 340 is formed in a columnar shape of which the diameter is made larger than the external diameter of the overtube body 320 using hard resins, metals, or the like, and a rear end surface thereof constitutes the base end surface 302 of the overtube 300. The base end cap 340 is provided with a through-hole 342 and a through-hole 344 that form a portion of the endoscope insertion passage 306 and a portion of the treatment tool insertion passage 308, respectively. In the base end surface 302, an opening of the through-hole 342 is equivalent to the above-described endoscope insertion port 310, and an opening of the through-hole 344 is equivalent to the above-described treatment tool insertion port 314.

Additionally, the through-holes 342 and 344 are provided with valve members 346 and 348. The valve members 346 and 348, for example, open in a case where the endoscope insertion part 102 and the treatment tool insertion part 202 are inserted therethrough and come into close contact with outer peripheral surfaces (side surfaces) of the endoscope insertion part 102 and the treatment tool insertion part 202 without a substantial gap. This secures the airtightness of spaces closer to the distal end side than the valve members 346 and 348, and reduces the leakage or the like of a pneumoperitoneum gas injected into the body cavity to the outside of the body.

The distal end cap 360 is formed of hard resins, metals, or the like, and a front end surface thereof constitutes the distal end surface 304 of the overtube 300. The distal end cap 360 is provided with a through-hole 362 and a through-hole 364 that form a portion of the endoscope insertion passage 306 and a portion of the treatment tool insertion passage 308, respectively. In the distal end surface 304, an opening of the through-hole 362 is equivalent to the above-described endoscope delivery port 312, and an opening of the through-hole 364 is equivalent to the treatment tool delivery port 316.

The above base end cap 340 and the above distal end cap 360 are some of the constituent elements of the overtube body of the invention, and may be formed separately from or formed integrally with the overtube body 320.

The slider 400 is housed within the cavity part 324 of the overtube body 320, and is supported so as to be movable forward and backward in the direction of the reference axis 300a. The slider 400 is an interlocking member that is coupled to the endoscope insertion part 102 inserted through the endoscope insertion passage 306 and the treatment tool insertion part 202 inserted through the treatment tool insertion passage 308 and that has a dead zone where the forward and backward movement of either the endoscope insertion part or the treatment tool insertion part in the forward-backward direction (axial direction) does not interlock with the movement of the other and a sensing zone where the forward and backward movement of either the endoscope insertion part or the treatment tool insertion part interlocks with the movement of the other. That is, the endoscope insertion part 102 is adapted to interlock with the forward and backward movement of the treatment tool insertion part 202 in the axial direction with play by the slider 400.

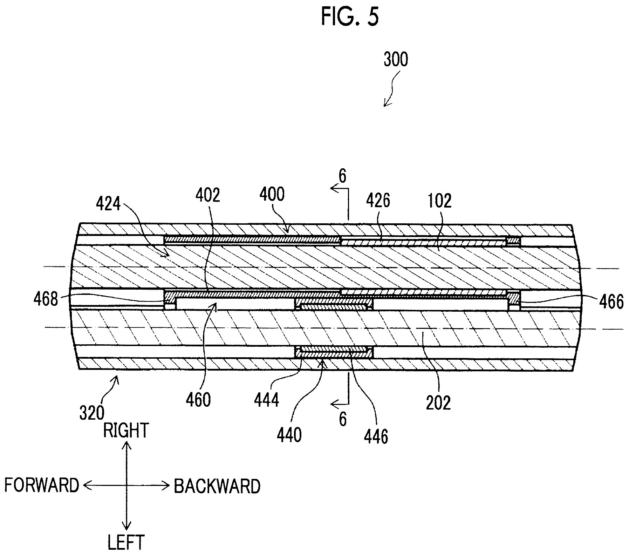

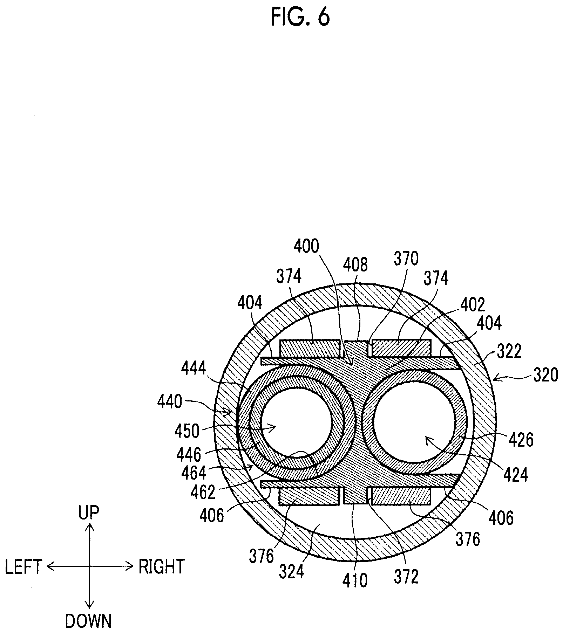

Fig. 5 is an enlarged sectional view illustrating a portion, in which the slider 400 is arranged in Fig. 4, in an enlarged manner, and illustrates a state where the endoscope insertion part 102 and the treatment tool insertion part 202 have been inserted through the endoscope insertion passage 306 and the treatment tool insertion passage 308, respectively. Fig. 6 is a sectional view when viewed from arrow 6-6 in Fig. 5.

As illustrated in Figs. 5 and 6, the slider 400 has a slider body 402 (slider member) that holds components of the slider 400. As illustrated in Fig. 6, protruding strips 408 and 410 that extend in the direction (forward-backward direction) of the reference axis 300a are formed on a flat upper surface 404 and a flat lower surface 406 of the slider body 402.

Meanwhile, a pair of left and right long plate-shaped guide plates 374 and 374 and a pair of left and right long plate-shaped guide plates 376 and 376, which are laid between the base end cap 340 and the distal end cap 360, are respectively supported by an upper part and a lower part within the cavity part 324 of the overtube body 320, and guide grooves 370 and 372, which extend in the direction of the reference axis 300a from the base end cap 340 to the distal end cap 360, are formed by a gap between the guide plates 374 and 374 and a gap between the guide plates 376 and 376.

The protruding strips 408 and 410 of the slider body 402 are respectively fitted into the guide grooves 370 and 372 within the cavity part 324, and the upper surface 404 and the lower surface 406 are arranged in a state where these surfaces have contacted or approached the guide plates 374 and 374 and the guide plates 376 and 376..

Accordingly, the slider 400 is supported so as to be movable forward and backward in the forward-backward direction within the cavity part 324, and is supported in a state where the movement of the slider in the upward-downward direction and in the leftward-rightward direction and the rotation of the slider in all directions (the rotation of the slider around three axes including a forward-backward axis, a leftward-rightward axis, and an upward-downward direction) are restricted. Additionally, the slider 400 moves forward and backward within a movable range having a position where the slider abuts against the base end cap 340 as a rear end, and having a position where the slider abuts against the distal end cap 360 as a front end.

In addition, the guide grooves 370 and 372 may not be formed by the guide plates 374 and 374 and the guide plates 376 and 376 arranged within the cavity part 324 of the overtube body 320, and may be formed in the outer wall 322 of the overtube body 320 or may be formed by other configurations.

Additionally, the slider 400, as illustrated in Fig. 4, has an endoscope-coupled part 420 that is coupled to (engaged with) the endoscope insertion part 102, and a treatment tool-coupled part 422 that is coupled to (engaged with) the treatment tool insertion part 202.

The endoscope-coupled part 420 is provided on the right side of the slider body 402, and includes a through-hole 424 (refer to Fig. 6) in which a space serving as the endoscope insertion passage 306 is secured within the cavity part 324 of the overtube body 320 and through which, as illustrated in Fig. 5, the endoscope insertion part 102 is inserted, and a pressure-contact member 426 that is fixed to the through-hole 424, is brought into pressure contact with the outer peripheral surface (side surface) of the endoscope insertion part 102 inserted through the endoscope insertion passage 306. The pressure-contact member 426 is annularly formed of elastic materials, such as elastic rubber, as illustrated in Fig. 6.

Accordingly, when the endoscope insertion part 102 has been inserted through the endoscope insertion passage 306, as illustrated in Fig. 5, the endoscope insertion part 102 is inserted through the through-hole 424, and the pressure-contact member 426 is brought into pressure contact with (engaged with) the outer peripheral surface of the endoscope insertion part 102. The endoscope insertion part 102 and the slider 400 (slider body 402) are coupled to (engaged with) each other in an interlockable manner via the pressure-contact member 426, and the slider 400 (slider body 402) also integrally moves forward and backward in an interlocking manner with the forward and backward movement of the endoscope insertion part 102 in the forward-backward direction (axial direction).

In addition, since the coupling herein is based on the elastic force of the pressure-contact member 426, the engagement position (the position of the endoscope insertion part 102 where the slider 400 is engaged) of the endoscope insertion part 102 coupled to the slider 400 (slider body 402) can be arbitrarily adjusted.

The treatment tool-coupled part 422, as illustrated in Fig. 4, is provided on the left side of the slider body 402, and as illustrated in Fig. 5, includes a sleeve 440 (sleeve member) that is coupled to the treatment tool insertion part 202, and a guide part 460 that guides the sleeve 440 so as to be movable forward and backward in the forward-backward direction.

The sleeve 440, as illustrated in Fig. 6, includes a sleeve body (frame body) 444 formed in a cylindrical shape, and a pressure-contact member 446 fixed to the inside of the sleeve body 444. The pressure-contact member 446 is annularly formed of elastic materials, such as elastic rubber.

Accordingly, when the treatment tool insertion part 202 has been inserted through the treatment tool insertion passage 308, as illustrated in Fig. 5, the treatment tool insertion part 202 is inserted through the inside (the through-hole 450 of Fig. 6) of the pressure-contact member 446, the pressure-contact member 446 is brought into pressure contact with (engaged with) the outer peripheral surface of the treatment tool insertion part 202. The treatment tool insertion part 202 and the sleeve 440 are coupled with each other in an interlockable manner via the pressure-contact member 446, and the sleeve 440 also integrally moves forward and backward in an interlocking manner with the forward and backward movement of the treatment tool insertion part 202 in the forward-backward direction (axial direction).

Additionally, the sleeve 440 also rotates with respect to the slider body 402 in an interlocking manner with the rotation of the treatment tool insertion part 202 around the axis thereof.

In addition, since the coupling between the treatment tool insertion part 202 and the sleeve 440 herein is based on the elastic force of the pressure-contact member 446, the engagement position (the position of the treatment tool insertion part 202 where the sleeve 440 is engaged) of the treatment tool insertion part 202 coupled to the sleeve 440 can be arbitrarily adjusted.

Meanwhile, the guide part 460 of the treatment tool-coupled part 422, as illustrated in Fig. 6, is formed by a space surrounded by a guide surface 462 of the slider body 402 that extends in the direction of the reference axis 300a within the cavity part 324 of the overtube body 320, and an inner peripheral surface of the overtube body 320. The sleeve 440 is housed and arranged in the space of the guide part 460, is supported so as to be movable in the forward-backward direction and rotatable around its axis, and is supported in a state where the movement of the sleeve in the upward-downward direction and in the leftward-rightward direction is restricted.

Additionally, the guide part 460 is provided so as to fall within a range from a base end of the slider body 402 to a distal end thereof, and as illustrated in Fig. 5, has end edge parts 466 and 468, which are formed to protrude in a direction orthogonal to the guide surface 462 along an end edge of the guide surface 462, respectively, on the base end side and the distal end side of the slider body 402.

The end edge parts 466 and 468 abut against the end of the sleeve 440 to restrict the movement of the sleeve 440, when the sleeve 440 arranged in the space of the guide part 460 moves forward and backward in the forward-backward direction.

Therefore, the sleeve 440 moves forward and backward within a movable range having a position where the sleeve abuts against the end edge part 466 as a rear end, and having a position where the sleeve abuts against the end edge part 468 as a front end. However, the rear end and the front end of the movable range of the sleeve 440 may not be restricted by the end edge part 466 and the end edge part 468.

According to the slider 400 configured as described above, the endoscope insertion part 102 inserted through the endoscope insertion passage 306 of the overtube 300 and the slider body 402 are coupled together, and the treatment tool insertion part 202 inserted through the treatment tool insertion passage 308 of the overtube 300 and the sleeve 440 are coupled together.

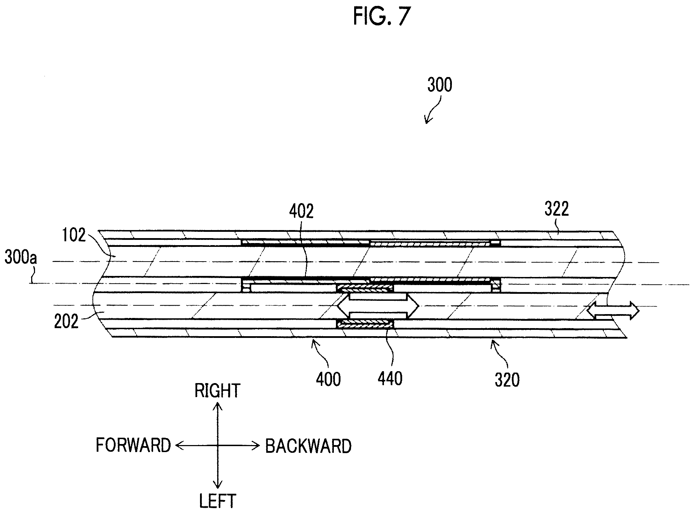

As illustrated in Fig. 7, it is supposed that a surgeon performs a forward and backward movement operation for moving the treatment tool insertion part 202 forward and backward in the axial direction (forward-backward direction) in a state where the sleeve 440 has not reached the rear end and the front end of the movable range thereof with respect to the slider body 402 (guide part 460).

In this case, in a case where the sleeve 440 has moved forward and backward within the movable range thereof with respect to the slider body 402, the slider body 402 does not move with respect to the forward and backward movement of the treatment tool insertion part 202. Therefore, a forward and backward movement operation in the dead zone where the endoscope insertion part 102 does not interlock with the forward and backward movement of the treatment tool insertion part 202 is performed.

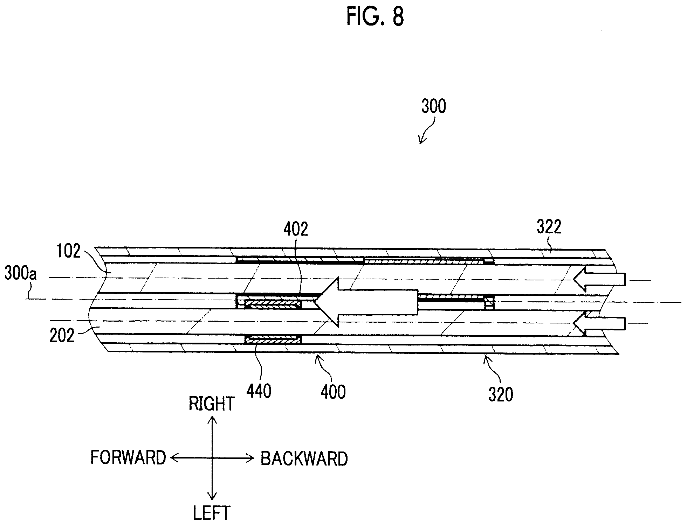

Meanwhile, as illustrated in Fig. 8, if the treatment tool insertion part 202 is operated to move forward in a state where the sleeve 440 reaches the front end of the movable range thereof with respect to the slider body 402, the sleeve 440 and the slider body 402 move forward with respect to the overtube body 320 together with the treatment tool insertion part 202. Accordingly, a forward and backward movement operation in the sensing zone where the endoscope insertion part 102 moves forward in an interlocking manner with the treatment tool insertion part 202 is performed.

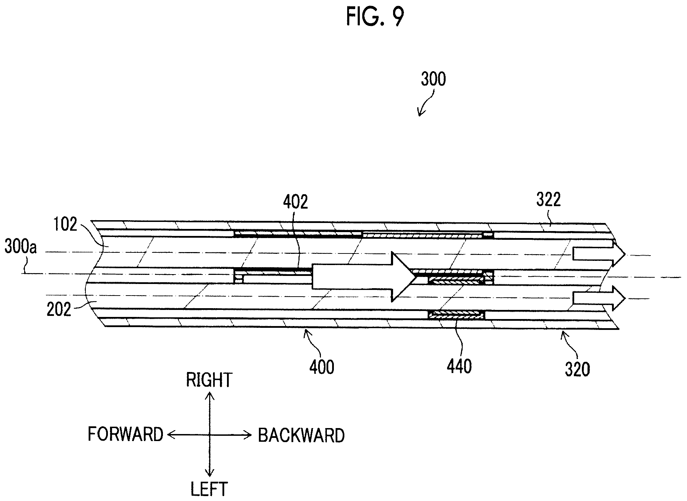

Similarly, as illustrated in Fig. 9, if the treatment tool insertion part 202 is operated to move backward in a state where the sleeve 440 reaches the rear end of the movable range thereof with respect to the slider body 402, the sleeve 440 and the slider body 402 move backward with respect to the overtube body 320 together with the treatment tool insertion part 202. Accordingly, a forward and backward movement operation in the sensing zone where the endoscope insertion part 102 moves backward in an interlocking manner with the treatment tool insertion part 202 is performed.

As described above, according to the slider 400 of the present embodiment, in a case where the forward and backward movement operation (the forward and backward movement operation in the sensing zone) of the treatment tool insertion part 202 is performed, the endoscope insertion part 102 is displaced in the axial direction in an interlocking manner with the treatment tool insertion part 202, and in a case where a small amplitude of forward and backward movement operation (the forward and backward movement operation in the dead zone) of the treatment tool insertion part 202 is performed, the endoscope insertion part 102 is not displaced in the axial direction.

Accordingly, in a case where a surgeon has operated to move the treatment tool insertion part 202 forward and backward in the axial direction, the endoscope insertion part 102 also moves in an interlocking manner forward, backward, upward, downward, rightward, and leftward when a large amplitude of forward and backward movement operation is performed. Thus, the visual field, orientation, and the like of the endoscope 100 can be changed as intended by the surgeon. Additionally, the visual field is always given to pick up an image of the distal end of the treatment tool 200 and consequently, an image that is optimal for treatment is automatically provided. In a case where it is desired to check sites other than a site to be treated, the checking can be performed by moving the treatment tool insertion part 202, and a surgeon can perform operations as desired. Therefore, an assistant (endoscopic technician) who operates the endoscope 100 apart from the surgeon can be made unnecessary, and a troublesome condition in which the surgeon should instruct an assistant about the visual field, orientation, and the like of the endoscope 100 serially can be eliminated.

Additionally, when a small amplitude of forward and backward movement operation of the treatment tool insertion part 202 has been performed, the endoscope insertion part 102 does not interlock. Therefore, the size of an object to be observed within an observation image can be prevented from fluctuating unnecessarily, a sense of perspective can be suitably maintained, and a stable observation image can be provided.

In addition, the sleeve 440 is rotatable with respect to the slider body 402 around its axis. Therefore, in a case where the treatment tool insertion part 202 has been operated to rotate around its axis, the treatment tool insertion part 202 can also be rotated around its axis together with the sleeve 440 without rotating the slider body 402 (without changing the positional relationship (position within the body cavity) of the endoscope insertion part 102 and the treatment tool insertion part 202 with respect to the overtube 300).

Next, the guide member 230 provided in the operating part 204 of the treatment tool 200 illustrated in Fig. 1 will be described.

As illustrated in Fig. 1, the operating part 204 of the treatment tool 200 and the cable part 104 of an endoscope 100 are arranged adjacent to each other, in a state where the endoscope 100 and the treatment tool 200 are respectively inserted through the endoscope insertion passage 306 and the treatment tool insertion passage 308 of the overtube 300, and the position of the distal end surface 114 of the endoscope 100 in the axial direction (forward-backward direction) is adjusted with respect to the position of the treatment part 206 of the treatment tool 200 so that the treatment part 206is reflected on an observation image.

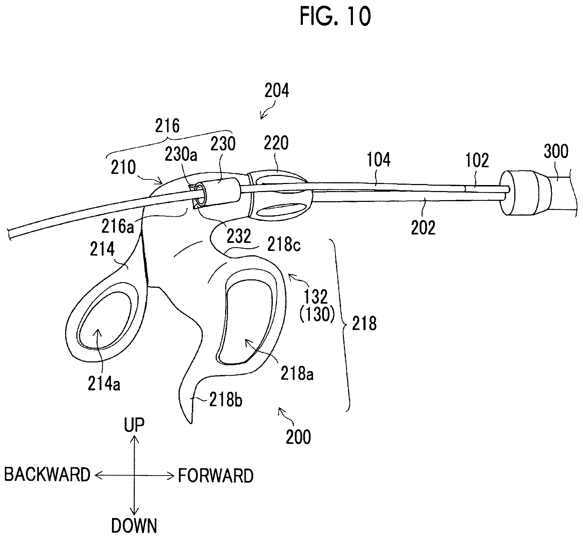

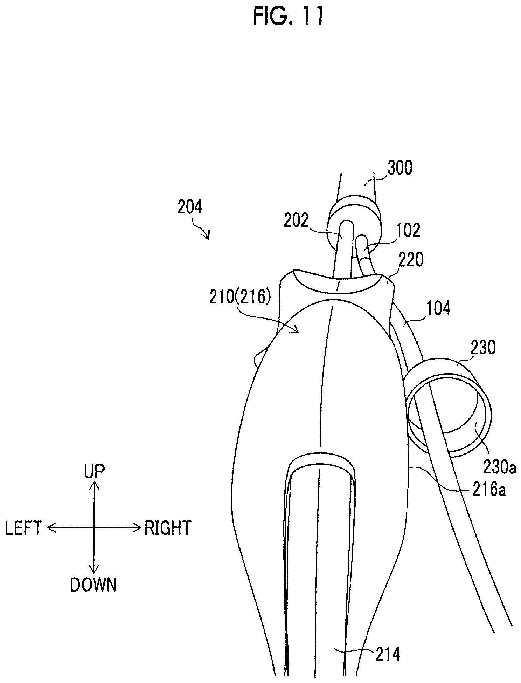

Fig. 10 and Fig. 11 are external views illustrating the operating part 204 of the treatment tool 200 from a right side and a rear side.

As illustrated in these drawings, the operating part 204 of the treatment tool 200 includes the fixed handle 210, the movable handle 214, the rotating handle 220, and the guide member 230. Since the actions of the movable handle 214 and the rotating handle 220 is as described above, the description thereof will be omitted herein.

The fixed handle 210 indicates a portion in which a body part 216 that houses internal parts of the operating part 204, and a handle part 218 for allowing a surgeon to grip the operating part 204 are integrally formed.

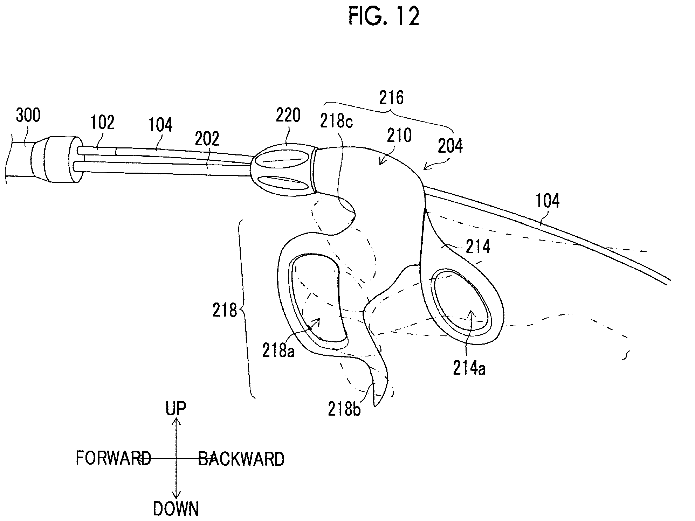

The handle part 218 includes a fixed ring part 218a having a hole for allowing a middle finger and a third finger of a right hand to be inserted therethrough in a case where a surgeon grips the operating part 204 with his/her right hand as illustrated in Fig. 12, a circular arc part 218b on which a little finger of the right hand is hooked, and a circular arc part 218c on which an index finger of the right hand is hooked.

The movable handle 214 includes a movable ring part 214a having a hole for allowing the thumb of the right hand to be inserted therethrough, in a case where the surgeon grips the operating part 204 with his/her right hand as illustrated in Fig. 12.

The guide member 230 is provided on a right side surface 216a of the body part 216 with respect to the configuration of such an operating part 204.

Here, the orientation of the operating part 204 of the treatment tool 200 in the leftward-rightward direction and in the upward-downward direction in a state where the treatment tool insertion part 202 is inserted through the treatment tool insertion passage 308 of the overtube 300 can be freely changed with respect to the overtube 300 with the rotation of the treatment tool insertion part 202 around its axis in the treatment tool insertion passage 308.

Therefore, a forward-backward direction, a leftward-rightward direction, and an upward-downward direction are defined with respect to the operating part 204 as illustrated in Figs. 10 and 11, apart from the forward-backward direction, the leftward-rightward direction, and the upward-downward direction that are defined to the overtube 300 as in Fig. 3 and the like. That is, in the operating part 204, a side where the treatment tool insertion part 202 (rotating handle 220) is formed when a direction (the axial direction of the treatment tool insertion part 202) in which the treatment tool insertion part 202 is provided to extend is the forward-backward direction), is defined as a front side of the operating part 204. A portion where the handle part 218 is provided when a direction which is orthogonal to the forward-backward direction and in which the handle part 218 is provided to extend with respect to the body part 216 is the upward-downward direction, is defined as a lower side of the operating part 204. Additionally, a right side (a portion where the guide member 230 is provided) in a front view in which the operating part 204 is viewed from the rear side when a direction orthogonal to the forward-backward direction and the leftward-rightward direction is the leftward-rightward direction is defined as a right side of the operating part 204.

The guide member 230 is formed in a cylindrical shape (ring shape), for example using elastic materials, such as elastic rubber, hard plastics, or metals, or the like, and has an insertion hole 230a with a larger diameter than at least the diameter of the cable part 104 of the endoscope 100. A portion of an outer peripheral surface of the guide member 230 is fixed to the right side surface 216a of the body part 216, and an axis (central axis) of the insertion hole 230a is arranged so as to face the forward-backward direction.

As fixing means for fixing the guide member 230 to the right side surface 216a of the body part 216, for example, a form in which a plate-shaped mounting part 232 is provided in a portion of an outer peripheral part of the guide member 230 through integral molding with the guide member 230 as illustrated in Fig. 10, and the mounting part 232 is fixed to the right side surface 216a, is possible. In this case, in Fig. 10, the mounting part 232 is fitted and fixed to a recess provided in the right side surface 216a. However, regardless of the presence/absence of the recess, the mounting part 232 may be fixed to the right side surface 216a using an adhesive or an adhesive tape, or may be fixed with a screw. Additionally, engagement means for being engaged with the mounting part 232 may be provided on the right side surface 216a so that the mounting part can be fixed by the engagement, or the fixing means for fixing the guide member 230 to the right side surface 216a of the body part 216 can have arbitrary forms.

In this way, the cable part 104 of the endoscope 100 is arranged so as to be inserted through the insertion hole 230a of the guide member 230 installed in the right side surface 216a of the body part 216 of the operating part 204, and the movement range of the cable part 104 of the endoscope 100 in the leftward-rightward direction and in the upward-downward direction is limited to the range of the insertion hole 230a of the guide member 230.

Therefore, the flexible cable part 104 is guided by the guide member 230 so as to be movable forward and backward in the axial direction, without being separated from the surface of the operating part 204 beyond a fixed distance. Accordingly, it is also possible to reliably perform the forward and backward movement operation of the endoscope insertion part 102 with a right hand griping the operating part 204 as will be described next. That is, it is possible to perform the operation of the treatment tool 200 and the forward and backward movement operation of the endoscope insertion part 102 with a single hand.

The action of the guide member 230 of the operating part 204 will be described.

As illustrated in Fig. 1, a state where the endoscope 100 (endoscope insertion part 102) and the treatment tool 200 (treatment tool insertion part 202) are respectively inserted through the endoscope insertion passage 306 and the treatment tool insertion passage 308 of the overtube 300, and the position of the distal end surface 114 of the endoscope 100 in the axial direction (forward-backward direction) is adjusted with respect to the position of the treatment part 206 of the treatment tool 200 so that the treatment part 206 is reflected on an observation image is brought about. In this state, the operating part 204 of the treatment tool 200 and the cable part 104 of the endoscope 100 are arranged in proximity with each other by achieving a reduction in diameter of the overtube 300.

In this case, a portion of the operating part 204 may overhang the axis of the endoscope insertion part 102 inserted through the endoscope insertion passage 306 of the overtube 300 depending on the size of the operating part 204 of the treatment tool 200. In this case, the cable part 104 may be brought into contact with the distal end (rotating handle 220) of the operating part 204, or the like and bent, and may be greatly separated from the operating part 204. Additionally, the invention is not limited to this case, and the cable part 104 may be bent due to its own weight or the like and may be greatly separated from the operating part 204. In this way, if the cable part 104 is greatly separated from the operating part 204, an index finger of a right hand gripping the operating part 204 does not reach the cable part 104, and the operation of the treatment tool 200 and the forward and backward movement operation of the endoscope insertion part 102 cannot be performed with a single hand.

Meanwhile, when the endoscope insertion part 102 of the endoscope 100 is inserted through the endoscope insertion passage 306 of the overtube 300, the cable part 104 of the endoscope 100 is inserted into and arranged in the guide member 230 as illustrated in Figs. 10 and 11 by inserting the endoscope insertion part 102 through the guide member 230 of the operating part 204, and then inserting the endoscope insertion part 102 through the endoscope insertion passage 306 of the overtube 300.

Accordingly, the flexible cable part 104 is always arranged within a range of a fixed distance from the operating part 204, that is, within a range where an index finger of a right hand gripping the operating part 204 reaches, without being separated from the operating part 204 due to bending.

Here, a state where the endoscope insertion part 102 of the endoscope 100 can be arranged on the right side of the operating part 204 and the cable part 104 through which the guide member 230 is inserted is smoothly movable forward and backward inside the insertion hole 230a of the guide member 230 is brought about by adjusting the relative orientation of the operating part 204 with respect to the overtube 300 so that the leftward-rightward direction and the upward-downward direction of the overtube 300 and the leftward-rightward direction and the upward-downward direction of the operating part 204 of the treatment tool 200 substantially coincide with each other. However, the leftward-rightward direction and the upward-downward direction of the overtube 300 and the leftward-rightward direction and the upward-downward direction of the operating part 204 of the treatment tool 200 may not necessarily coincide with each other.

As illustrated in Fig. 12, a surgeon usually grips the operating part 204 of the treatment tool 200 with his/her right hand to perform a required operation, the middle finger and the third finger of his/her right hand are inserted into a fixed ring part 218a of the fixed handle 210 (handle part 218), and the thumb is inserted through a movable ring part 214a of the movable handle 214. The little finger is hooked on a circular arc part 218b of the handle part 218, and the index finger is hooked on a circular arc part 218c of the handle part 218.

In a case where the gripping members of the treatment part 206 are opened and closed and in a case where the surgeon's thumb is moved forward and backward to move the movable handle 214 forward and backward and rotate the gripping members of the treatment part 206, the index finger is released from the circular arc part 218c and is made to abut against the side surface of the front rotating handle 220, and the index finger is moved in a desired rotational direction to rotate the rotating handle 220.

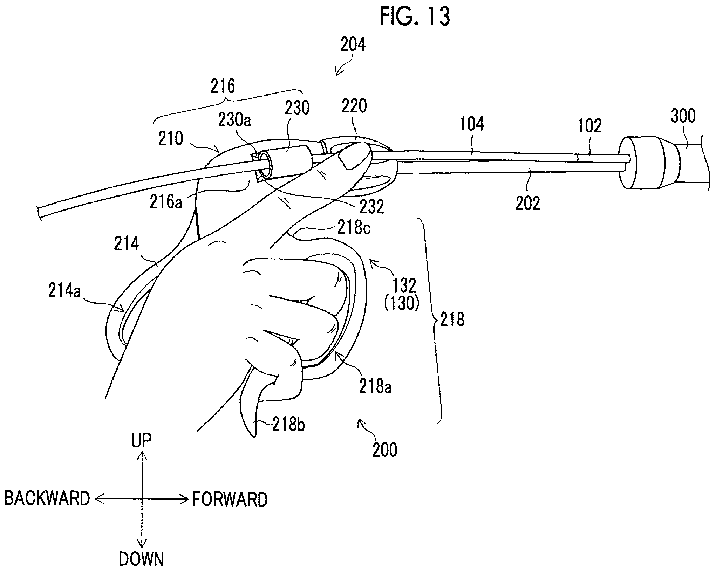

Since the index finger of the right hand can be relatively freely moved in a state where the operating part 204 of the treatment tool 200 is gripped with the right hand in this way, the cable part 104 can be easily sandwiched between the index finger and the operating part 204 as illustrated in Fig. 13, by using the index finger. That is, the cable part 104 can be held down with the index finger of the right finger.

A recess that extends in the forward-backward direction for facilitating hooking of a finger is formed in the rotating handle 220 of the present embodiment, and in the example illustrated in Fig. 13, the cable part 104 is held down in the recess. However, the position where the cable part 104 is held down may not necessarily be the position of the rotating handle 220, and just has to hold the cable part down at an arbitrary position of the operating part 204.

Accordingly, a state where the forward and backward movement operation of the endoscope 100 is possible only with the right hand without separating the right hand gripping the treatment tool 200 from the treatment tool 200 is brought about.

Then, by bending or extending the index finger while holding down the cable part 104 in a state where the cable part 104 is held down with the index finger in this way, the cable part 104 can be made to slide in the forward-backward direction with respect to the operating part 204, and only the endoscope 100 can be moved forward and backward in the forward-backward direction with respect to the treatment tool 200. Accordingly, it is possible to perform change to a visual field suitable for detailed observation and treatment, and surgical efficiency can be improved.

Additionally, if the overall right hand is moved in the forward-backward direction without moving the index finger holding down the cable part 104 to move the treatment tool 200 forward and backward in the forward-backward direction, it is also possible to forcibly invalidate the action of the dead zone of the slider 400 of the overtube 300 to move the endoscope 100 forward and backward together with the treatment tool 200.

Next, an example of the forward and backward movement operation of the endoscope 100 and the treatment tool 200 in the endoscopic surgical device 10 of the present embodiment will be described.

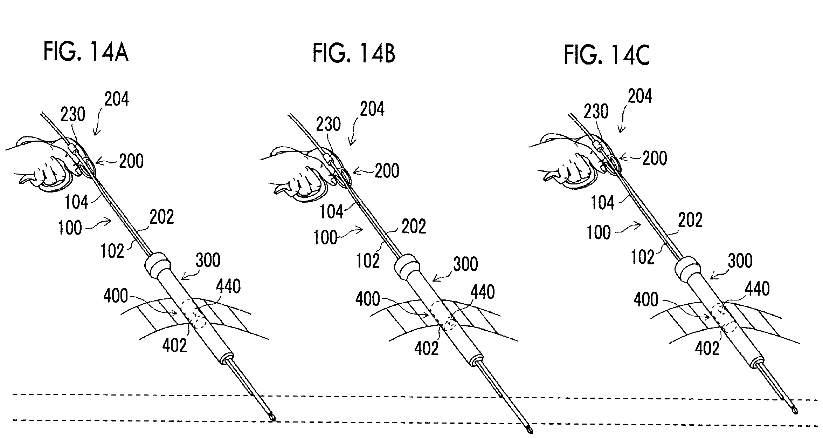

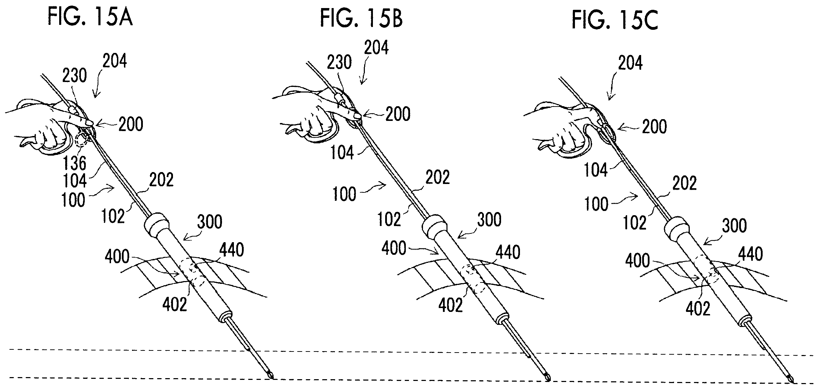

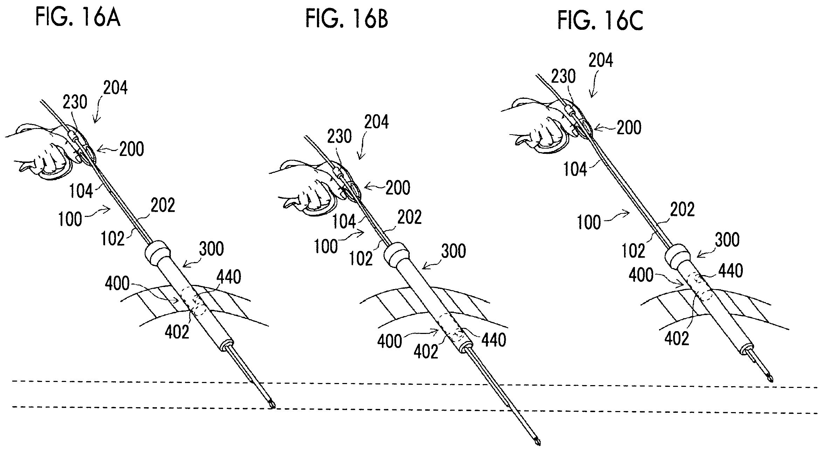

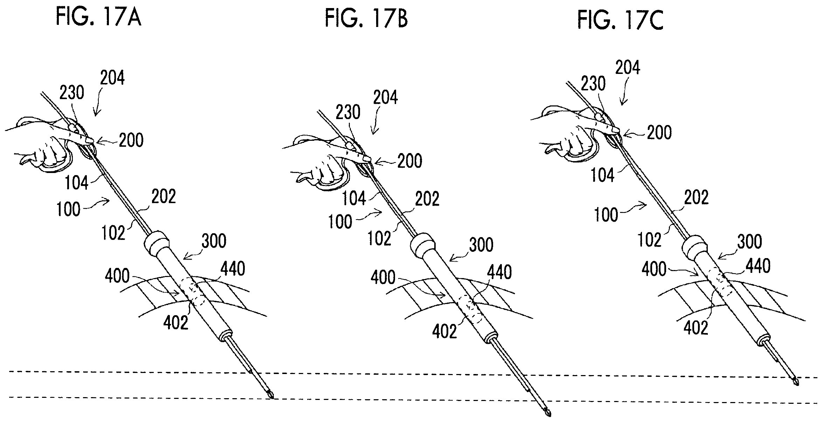

Figs. 14A to 17C are explanatory views illustrating the aspect of the operation when treatment of a diseased site within a patient's body cavity is performed using the endoscopic surgical device 10 of the present embodiment, Figs. 14A to 14C illustrate an aspect of the operation when only the treatment tool 200 moves forward and backward, Figs. 15A to 15C illustrate an aspect of the operation when only the endoscope 100 moves forward and backward, Figs. 16A to 16C illustrate an aspect of the operation when the endoscope 100 moves forward and backward in an interlocking manner with the treatment tool 200 through an interlocking function of the slider 400, and Figs. 17A to 17C illustrate an aspect of the operation when the endoscope 100 moves forward and backward in an interlocking manner with the treatment tool 200 through the interlocking function of the slider 400.

As illustrated in Fig. 14A, the endoscope 100 (endoscope insertion part 102) and the treatment tool 200 (treatment tool insertion part 202) are respectively inserted into the endoscope insertion passage 306 and the treatment tool insertion passage 308 of the overtube 300 after the overtube 300 is inserted into a patient's body wall and a pneumoperitoneum gas is injected into a body cavity. In this case, the endoscope 100 is coupled to the slider body 402 of the slider 400, and the treatment tool 200 is coupled to the sleeve 440 of the slider 400. Thus, when the sleeve 440 moves within a movable range thereof with respect to the slider body 402, the interlocking is performed with the dead zone (play) where the endoscope 100 does not interlock with the forward and backward movement of the treatment tool 200.

In this state, if the surgeon grips only the operating part 204 of the treatment tool 200 and minutely moves only the treatment tool 200 forward without holding down the cable part 104 of the endoscope 100, only the treatment tool 200 can be moved forward in a state where the endoscope 100 is made stationary as illustrated in Fig. 14B, with respect to the forward movement in the dead zone until the sleeve 440 of the slider 400 abuts against the front end of the movable range thereof.

Similarly, if the surgeon grips only the operating part 204 of the treatment tool 200 and minutely moves only the treatment tool 200 backward, only the treatment tool 200 can be moved backward in a state where the endoscope 100 is made stationary as illustrated in Fig. 14C, with respect to the backward movement in the dead zone until the sleeve 440 of the slider 400 abuts against the rear end of the movable range thereof.

Therefore, since the endoscope 100 does not move forward and backward with respect to the minute forward and backward movement operation of the treatment tool 200, the range of an observation image displayed on the monitor 112 does not change, the size of an object to be observed can be prevented from fluctuating according to the minute displacement of the treatment tool 200, a sense of perspective can be suitably maintained, and a stable observation image can be obtained.

Fig. 15A illustrates that the overtube 300, the endoscope 100, and the treatment tool 200 are in the same state as those of Fig. 14A.

In this state, if the surgeon sandwiches the cable part 104 of the endoscope 100 between the index finger of his/her right hand gripping the operating part 204 of the treatment tool 200 and the operating part 204 of the treatment tool 200, holds down the cable part 104, and moves the index finger forward (for example extends the index finger), only the endoscope 100 can be moved forward in a state where the treatment tool 200 is made stationary as illustrated in Fig. 15B, with respect to the forward movement in the dead zone until the sleeve 440 of the slider 400 abuts against the rear end of the movable range thereof.

Similarly, if the surgeon sandwiches the cable part 104 of the endoscope 100 between the index finger of his/her right hand gripping the operating part 204 of the treatment tool 200 and the operating part 204 of the treatment tool 200, holds down the cable part 104,and moves the index finger backward (for example bends the index finger), only the endoscope 100 can be moved backward in a state where the treatment tool 200 is made stationary as illustrated in Fig. 15C, with respect to the backward movement in the dead zone until the sleeve 440 of the slider 400 abuts against the front end of the movable range thereof.

Therefore, since the position of the distal end surface 114 of the endoscope 100 can be moved forward and backward without the treatment tool 200 moving forward and backward with respect to the forward and backward movement of the endoscopes 100, only the visual field (observation range) of the endoscope 100 can be changed. That is, the observation range can be magnified or diminished (the size of an object to be observed in the observation image is increased or reduced).

In addition, in order to facilitate the operation in a case where the endoscope 100 is moved forward and backward with respect to the treatment tool 200 as illustrated in Fig. 15A to Fig. 15B or 15C, as illustrated by a dashed line of Fig. 15A, the cable part 104 of the endoscope 100 may be provided with an annular ring member 136 that allows the index finger or the like to pass therethrough so as to perform the forward and backward movement operation of the endoscope 100 or an arbitrary-shaped finger-hooking part that allows a finger to be hooked thereon. In this case, it is desirable that the ring member 136 is provided so as to be rotatable in a direction around an axis of the cable part 104 and to make the orientation of the ring member 136 changeable. Additionally, it is more desirable to make the installation position of the ring member 136 changeable in the axial direction of the cable part 104. Moreover, the finger-hooking part like the ring member 136 may be detached from the cable part 104 for the sake of an unnecessary case.

Fig. 16A illustrates that the overtube 300, the endoscope 100, and the treatment tool 200 are in the same state as those of Fig. 14A.

In this state, if the surgeon grips only the operating part 204 of the treatment tool 200 without holding down the cable part 104 of the endoscope 100 and greatly moves the treatment tool 200 forward, the endoscope 100 can be moved forward in an interlocking manner with the forward movement of the treatment tool 200 through the interlocking function of the slider 400 as illustrated in Fig. 16B, after the forward movement in the dead zone until the sleeve 440 of the slider 400 abuts against the front end of the movable range thereof.

Similarly, if the surgeon grips only the operating part 204 of the treatment tool 200 and greatly moves the treatment tool 200 backward, the endoscope 100 can be moved backward in an interlocking manner with the backward movement of the treatment tool 200 through the interlocking function of the slider 400 as illustrated in Fig. 16C, after the backward movement in the dead zone until the sleeve 440 of the slider 400 abuts against the rear end of the movable range thereof.

Therefore, since the endoscope 100 moves forward and backward with respect to the forward and backward movement operation of the treatment tool 200, the range of an observation image displayed on the monitor 112 is continuously changed so as to follow the forward and backward movement of the treatment tool 200. Accordingly, since the size of an object to be observed changes according to the operation of the treatment tool 200, an image desired by a surgeon can be simply obtained.

Fig. 17A illustrates that the overtube 300, the endoscope 100, and the treatment tool 200 are in the same state as those of Fig. 14A.

In this state, if the surgeon sandwiches the cable part 104 of the endoscope 100 between the index finger of his/her right hand gripping the operating part 204 of the treatment tool 200 and the operating part 204 of the treatment tool 200, holds down the cable part 104, and moves the entire right hand forward, the endoscope 100 can be moved forward together with the treatment tool 200 as illustrated in Fig. 17B even in a state where the sleeve 440 of the slider 400 does not abuts against the front end of the movable range thereof.

Similarly, if the surgeon sandwiches the cable part 104 of the endoscope 100 between the index finger of his/her right hand gripping the operating part 204 of the treatment tool 200 and the operating part 204 of the treatment tool 200, holds down the cable part 104, and moves the entire right hand backward, the endoscope 100 can be moved backward together with the treatment tool 200 as illustrated in Fig. 17C even in a state where the sleeve 440 of the slider 400 does not abuts against the rear end of the movable range thereof.

Therefore, the dead zone of the slider 400 with which the endoscope 100 does not interlock can be invalidated with respect to the forward and backward movement of the treatment tools 200 and the endoscope 100. Thus, the endoscope 100 can be moved forward and backward more immediately than the forward and backward movement of the endoscope 100 through the interlocking function of the slider 400 described in Figs. 16A to 16C. In a case where the surgeon tries to immediately move the endoscope 100 forward and backward together with the treatment tool 200, the dead zone can be invalidated by selecting such an operation.

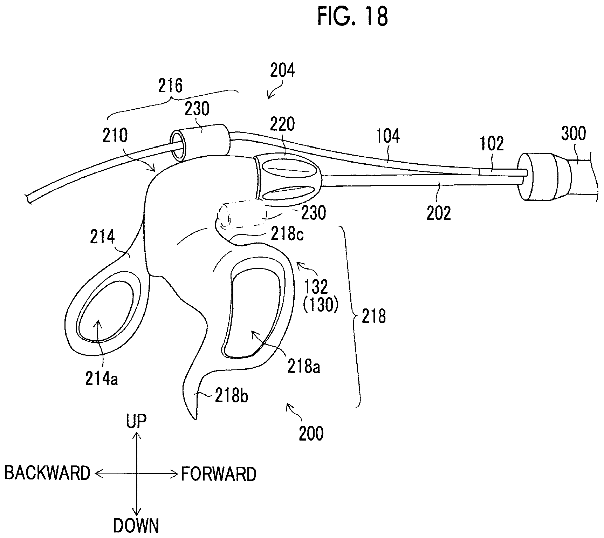

As described above, in the above embodiment, as illustrated in Figs. 10 and 11, the guide member 230 of the operating part 204 of the treatment tool 200 is provided on the right side surface 216a of the body part 216. However, the guide member 230 may be arranged on any of surfaces on an upper side, a lower side, a left side, and a right side of the operating part 204. For example, as illustrated in Fig. 18, the guide member 230 may be provided in an upper part (upper surface 216b) of the body part 216. Additionally, as illustrated by an imaginary line of this drawing, the guide member may be provided in a lower part of the body part 216. Additionally, a case where the operating part 204 of the treatment tool 200 is gripped with the right hand has been described in the above embodiment. However, in a case where the cable part 104 is gripped and held down with an index finger of a left hand, the guide member may be provided on a left end surface opposite to the right side surface 216a of the body part 216. In this case, the cable part 104 of the endoscope 100 can be easily arranged on the left side of the operating part 204 by adjusting the orientation of the operating part 204 with respect to the overtube 300 so that a downward direction of the operating part 204 becomes an upward direction of the overtube 300 illustrated in Fig. 3 and the like. Accordingly, a state where the cable part 104 through which the guide member 230 is inserted is smoothly movable forward and backward inside the insertion hole 230a can be brought about, and the cable part 104 can be held down with an index finger of a left finger at the position of a left side surface of the operating part 204.

Additionally, in the above embodiment, the guide member 230 is formed in a cylindrical shape. However, the shape of the guide member (the shape of the insertion hole 230a) in a section orthogonal to the axial direction may not necessarily have a circular shape, and may have arbitrary shapes, such as a quadrangular shape.

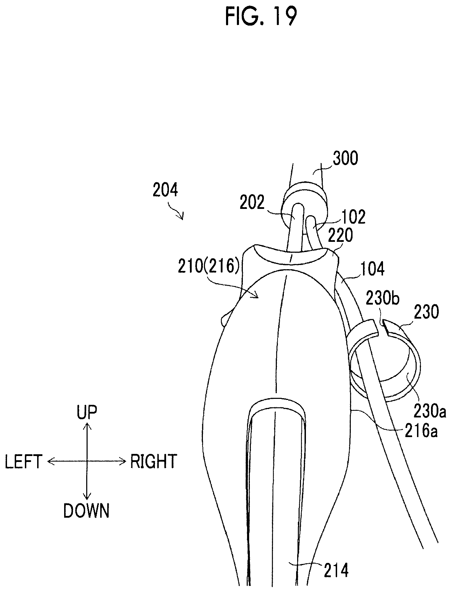

Additionally, in the above embodiment, in a case where the cable part 104 of the endoscope 100 is inserted into and arranged in the insertion hole 230a of the guide member 230, the cable part is inserted through the insertion hole 230a of the guide member 230 from the distal end side of the endoscope insertion part 102. However, the cable part may be inserted from a connector side of the cable part 104. Additionally, as illustrated in Fig. 19, a portion of the guide member 230 in the circumferential direction may be provided with a gap part 230b that extends in the axial direction. The gap part 230b is formed to pass through the guide member 230 from an outer peripheral surface of the guide member to an inner peripheral surface thereof, so that the cable part 104 of the endoscope 100 can be directly inserted into the inside of the insertion hole 230a of the guide member 230 from the gap part 230b.

Additionally, the overtube 300 of the above embodiment includes an interlocking mechanism that interlocks the endoscope 100 (endoscope insertion part 102) and the treatment tool 200 (treatment tool insertion part 202), which are respectively inserted through the endoscope insertion passage 306 and the treatment tool insertion passage 308 of the overtube 300, with each other with play using the slider 400. However, the invention is effective also in a case where an overtube, which includes an endoscope insertion passage through which the endoscope 100 (endoscope insertion part 102) is inserted and a treatment tool insertion passage through which the treatment tool 200 (treatment tool insertion part 202) is inserted, does not include an interlocking mechanism having play unlike the slider 400, and allows the endoscope 100 and the treatment tool 200 to move forward and backward in an interlocking manner with each other, is used. That is, only the endoscope 100 can be moved forward and backward only with the right hand, which grips the operating part 204 of the treatment tool 200, by the operation as illustrated in Figs. 15A to 15C, and the endoscope 100 can be moved forward and backward together with the treatment tool 200 only with the right hand, which grips the operating part 204 of the treatment tool 200, by the operation as illustrated in Figs. 17A to 17C.

### Explanation of References

- 10:: endoscopic surgical device
- 100:: endoscope
- 102:: endoscope insertion part
- 104:: cable part
- 108:: processor device
- 110:: light source device
- 112:: monitor
- 200:: treatment tool
- 202:: treatment tool insertion part
- 204:: operating part
- 206:: treatment part
- 210:: fixed handle
- 210a, 218a:: fixed ring part
- 210b, 210c, 218b, 218c:: circular arc part
- 214:: movable handle
- 214a:: movable ring part
- 216:: body part
- 216a:: right side surface
- 218:: handle part
- 220:: rotating handle
- 230:: guide member
- 230a:: insertion hole
- 230b:: gap part
- 232:: mounting part
- 300:: overtube
- 300a:: reference axis
- 302:: base end surface
- 306:: endoscope insertion passage
- 308:: treatment tool insertion passage
- 310:: endoscope insertion port
- 312:: endoscope delivery port
- 314:: treatment tool insertion port
- 316:: treatment tool delivery port
- 320:: overtube body
- 400:: slider
- 402:: slider body
- 420:: endoscope-coupled part
- 422:: treatment tool-coupled part
- 426,446:: pressure-contact member
- 440:: sleeve
- 444:: sleeve body

## Claims

1. A treatment tool (200) adapted to be used in an endoscopic surgical device including an endoscope (100), the treatment tool (200) and an overtube (300), wherein the endoscope (100) includes an endoscope insertion part (102) having an observation part (116) provided at a distal end thereof and a flexible cable (104) connected to a base end of the endoscope insertion part (102), wherein the treatment tool (200) includes a treatment tool insertion part (202) having a treatment part (206) provided at a distal end thereof and an operating part (204) for operating the treatment part (206) provided at a base end of the treatment tool insertion part (202), wherein the overtube (300) is for guiding the endoscope insertion part (102) and the treatment tool insertion part (202) into a body cavity and wherein the overtube (300) includes an overtube body (320) that passes through a body wall and is inserted into the body cavity, an endoscope insertion passage (306) that is provided inside the overtube body (320) and allows the endoscope insertion part (102) to be inserted therethrough so as to be movable forward and backward, and a treatment tool insertion passage (308) that is provided inside the overtube body (320) and allows the treatment tool insertion part (202) to be inserted therethrough so as to be movable forward and backward,
**characterized in that**
the operating part (204) is provided with a guide member (230) wherein the guide member (230) has an insertion hole with a diameter larger than a diameter of the flexible cable (104) and wherein the guide member (230) guides said flexible cable (104) so as to be movable forward and backward in an axial direction with respect to the operating part (204) so that the flexible cable (104) is kept from being separated from a surface of the operating part (204) beyond a fixed distance.

2. The treatment tool (200) according to claim 1,
wherein the guide member (230) is provided at a position where the flexible cable (104) is capable of being held down by an index finger of a hand operating the operating part (204).

3. The treatment tool (200) according to claim 1 or 2,
wherein the operating part (204) includes a handle part (210, 214) that is gripped by an operator and allows the operator to operate the treatment tool (200), and
wherein in a plan view in which the operating part (204) is viewed from a side opposite to a side where the treatment tool insertion part (202) is formed in an axial direction of the treatment tool insertion part (202), the guide member (230) is arranged on any of surfaces on an upper side, a lower side, a left side, and a right side of the operating part (204) in a state where the handle part (210, 214) is turned down.

4. The treatment tool (200) according to claim 3,
wherein the guide member (230) is arranged on a surface on a right side or a left side of the operating part (204).

5. An endoscopic surgical device (10) comprising:
the treatment tool (200) according to any one of claims 1 to 4; an endoscope (100) wherein the endoscope (100) includes an endoscope insertion part (102) having an observation part (116) provided at a distal end thereof and a flexible cable (104) connected to a base end of the endoscope insertion part; and an overtube (300) wherein the overtube (300) is for guiding the endoscope insertion part (102) and the treatment tool insertion part (202) into a body cavity and wherein the overtube (300) includes an overtube body (320) that passes through a body wall and is inserted into the body cavity, an endoscope insertion passage (306) that is provided inside the overtube body (320) and allows the endoscope insertion part (102) to be inserted therethrough so as to be movable forward and backward, and a treatment tool insertion passage (308) that is provided inside the overtube body (320) and allows the treatment tool insertion part (202) to be inserted therethrough so as to be movable forward and backward.

6. The endoscope surgical device (10) according to claim 5, further comprising:
an interlocking member (400) including an endoscope-coupled part (420) coupled to the endoscope insertion part (102) inserted through the endoscope insertion passage (306) and a treatment tool-coupled part (422) coupled to the treatment tool insertion part (202) inserted through the treatment tool insertion passage (308), the interlocking member (400) being arranged inside the overtube body (320) so as to be movable forward and backward,
wherein the interlocking member (400) includes a dead zone where the forward and backward movement of either the endoscope (100) or the treatment tool (200) does not interlock with the movement of the other and a sensing zone where the forward and backward movement of either the endoscope (100) or the treatment tool (200) interlocks with the movement of the other.

## Patentansprüche

1. Behandlungswerkzeug (200), ausgebildet zur Verwendung in einem endoskopischen chirurgischen Gerät, das ein Endoskop (100), das Behandlungswerkzeug (200) und ein Überrohr (300) enthält, von denen das Endoskop (100) einen Endoskopeinführteil (102) mit einem Betrachtungsteil (116) an seinem distalen Ende und ein mit einem Basisende des Endoskopeinführteils (102) verbundenes flexibles Kabel (104) enthält, wobei das Behandlungswerkzeug (200) einen Behandlungswerkzeug-Einführteil (202) mit einem an seinem distalen Ende befindlichen Behandlungsteil (206) und einem Bedienteil (204) zum Bedienen des Behandlungsteils (206) an einem Basisende des Behandlungswerkzeug-Einführteils (202) enthält, wobei das Überrohr (300) zum Führen des Endoskopeinführteils (102) und des Behandlungswerkzeug-Einführteils (202) in einen Körperhohlraum vorgesehen ist und das Überrohr (300) einen Überrohrkörper (320), der durch eine Körperwand hindurchtritt und in den Körperhohlraum eingeführt wird, einen Endoskopeinführkanal (306) im Inneren des Überrohrkörpers (320) zum Ermöglichen des durch ihn hindurchgehenden Eintritts des Endoskopeinführteils (102) zum Ausführen einer Bewegung nach vorn und nach hinten, und einen Behandlungswerkzeug-Einführkanal (308) im Inneren des Überrohrkörpers (320) zum Ermöglichen des Einführens des Behandlungswerkzeug-Einführteils (202) durch ihn hindurch für eine Vorwärts- und Rückwärtsbewegung enthält,
**dadurch gekennzeichnet, dass**
der Bedienteil (204) mit einem Führungselement (230) ausgestattet ist, welches ein Einführloch mit einem Durchmesser größer als der Durchmesser des flexiblen Kabels (104) aufweist, und das Führungselement (230) das flexible Kabel (104) derart führt, dass dieses in axialer Richtung bezüglich des Bedienteils (204) vorwärts und rückwärts bewegbar ist, so dass das flexible Kabel (104) von einer Oberfläche des Bedienteils (204) jenseits eines fixen Abstands getrennt gehalten wird.

2. Behandlungswerkzeug (200) nach Anspruch 1,
bei dem das Führungselement (230) an einer Stelle vorgesehen ist, an der das flexible Kabel (104) von einem Zeigefinger einer den Bedienteil bedienenden Hand niedergehalten werden kann.

3. Behandlungswerkzeug (200) nach Anspruch 1 oder 2,
bei dem der Bedienteil (204) einen Handgriff (210, 214) enthält, der von einem Operateur ergriffen wird und diesem gestattet, das Behandlungswerkzeug (200) zu bedienen, und
wobei in einer Draufsicht, in welcher der Bedienteil (204) von einer Seite entgegengesetzt zu einer Seite betrachtet wird, auf der der Behandlungswerkzeug-Einführteil (202) in axialer Richtung des Behandlungswerkzeug-Einführteils (202) gebildet ist, das Führungselement (230) an irgendeiner der Oberflächen auf einer Oberseite, einer Unterseite, einer linken Seite und einer rechten Seite des Bedienteils (204) in einem Zustand angeordnet ist, in welchem der Handgriff (210, 214) nach unten verdreht ist.

4. Behandlungswerkzeug (200) nach Anspruch 3,
bei dem das Führungselement (230) auf einer Oberfläche auf einer rechten Seite oder einer linken Seite des Bedienteils (204) angeordnet ist.

5. Endoskopisches chirurgisches Gerät (10), umfassend:
ein Behandlungswerkzeug (200) nach einem der Ansprüche 1 bis 4; ein Endoskop (100), welches einen Endoskopeinführteil (102) mit einem Betrachtungsteil (116) an seinem distalen Ende und ein mit einem Basisende des Endoskopeinführteils verbundenes flexibles Kabel (104) enthält; und ein Überrohr (300), das zum Führen des Endoskopeinführteils (102) und des Behandlungswerkzeug-Einführteils (202) in einen Körperhohlraum dient, wobei das Überrohr (300) einen Überrohrkörper (320) enthält, der durch eine Körperwand hindurchgeht und in den Körperhohlraum eingeführt wird, einen Endoskopeinführkanal (306), der sich im Inneren des Überrohrkörpers (320) befindet und ermöglicht, dass der Endoskopeinführteil (102) durch ihn hindurch vorwärts und rückwärts bewegbar eingeführt werden kann, und einen Behandlungswerkzeug-Einführkanal (308), der im Inneren des Überrohrkörpers (320) vorgesehen ist und dem Behandlungswerkzeug-Einführteil (202) ermöglicht, durch ihn hindurch vorwärts und rückwärts bewegbar eingeführt zu werden.

6. Endoskopisches chirurgisches Gerät (10) nach Anspruch 5, weiterhin umfassend:
ein Sperrelement (400) mit einem mit dem Endoskop gekoppelten Teil (420), der an den Endoskopeinführteil (102) gekoppelt ist, welcher durch den Endoskopeinführkanal (306) eingeführt ist, und mit einem an ein Behandlungswerkzeug gekoppelten Teil (422), der an den durch den Behandlungswerkzeug-Einführkanal (308) eingeführten Behandlungswerkzeug-Einführteil (202) gekoppelt ist, wobei das Sperrelement (400) im Inneren des Überrohrkörpers (320) vorwärts und rückwärts bewegbar angeordnet ist,
wobei das Sperrelement (400) eine Totzone enthält, in welcher die Vorwärts- und Rückwärtsbewegung von entweder dem Endoskop (100) oder dem Behandlungswerkzeug (200) sich nicht mit der Bewegung des anderen Teils verriegelt, und eine Sensorzone enthält, in der die Vorwärts- und Rückwärtsbewegung entweder des Endoskops (100) der des Behandlungswerkzeugs (200) sich mit der Bewegung des anderen Teils verriegelt.

## Revendications

1. Outil de traitement (200) apte à être utilisé dans un dispositif chirurgical endoscopique incluant un endoscope (100), l'outil de traitement (200) et un surtube (300), dans lequel l'endoscope (100) inclut une partie d'introduction d'endoscope (102) présentant une partie d'observation (116) prévue sur une extrémité distale de celle-ci et un câble flexible (104) raccordé à une extrémité de base de la partie d'introduction d'endoscope (102) ; dans lequel l'outil de traitement (200) inclut une partie d'introduction d'outil de traitement (202) présentant une partie de traitement (206) prévue sur une extrémité distale de celle-ci et une partie de manœuvre (204) pour manœuvrer la partie de traitement (206) prévue sur une extrémité de base de la partie d'introduction d'outil de traitement (202) ; dans lequel le surtube (300) est destiné à guider la partie d'introduction d'endoscope (102) et la partie d'introduction d'outil de traitement (202) dans une cavité corporelle, et dans lequel le surtube (300) inclut un corps de surtube (320), lequel passe à travers une paroi corporelle et est introduit dans la cavité corporelle, un passage pour introduction d'endoscope (306), lequel est prévu dans le corps de surtube (320) et permet l'introduction de la partie d'introduction d'endoscope (102) à travers lui de manière à ce que celle-ci puisse se déplacer vers l'avant et l'arrière, et un passage pour introduction d'outil de traitement (308), lequel est prévu dans le corps de surtube (320) et permet l'introduction de la partie d'introduction d'outil de traitement (202) à travers lui de manière à ce que celle-ci puisse se déplacer vers l'avant et l'arrière,
**caractérisé en ce que** la partie de manœuvre (204) est dotée d'un élément de guidage (230), dans lequel l'élément de guidage (230) présente un trou d'introduction avec un diamètre plus grand qu'un diamètre du câble flexible (104), et dans lequel l'élément de guidage (230) guide ledit câble flexible (104) de manière à ce qu'il puisse se déplacer vers l'avant et l'arrière dans une direction axiale par rapport à la partie de manœuvre (204), de sorte que le câble flexible (104) est maintenu séparé d'une surface de la partie de manœuvre (204) au-delà d'une distance fixe.

2. Outil de traitement (200) selon la revendication 1,
dans lequel l'élément de guidage (230) est prévu sur une position où le câble flexible (104) est capable d'être maintenu en bas par un index d'une main manœuvrant la partie de manœuvre (204).

3. Outil de traitement (200) selon la revendication 1 ou 2,
dans lequel la partie de manœuvre (204) inclut une partie formant poignée (210, 214), laquelle est saisie par un opérateur et permet à l'opérateur de manœuvrer l'outil de traitement (200), et
dans lequel dans une vue du dessus, où la partie de manœuvre (204) est vue à partir d'un côté opposé à un côté où la partie d'introduction d'outil de traitement (202) est formée dans une direction axiale de la partie d'introduction d'outil de traitement (202), l'élément de guidage (230) est agencé sur une surface quelconque parmi des surfaces sur un côté supérieur, un coté inférieur, un côté gauche, et un côté droit de la partie de manœuvre (204) dans un état où la partie formant poignée (210, 214) est rabattue.

4. Outil de traitement (200) selon la revendication 3,
dans lequel l'élément de guidage (230) est agencé sur une surface sur un côté droit ou un côté gauche de la partie de manœuvre (204).

5. Dispositif chirurgical endoscopique (10), comprenant :
l'outil de traitement (200) selon l'une quelconque des revendications 1 à 4 ; un endoscope (100), dans lequel l'endoscope (100) inclut une partie d'introduction d'endoscope (102) présentant une partie d'observation (116) prévue sur une extrémité distale de celle-ci et un câble flexible (104) raccordé à une extrémité de base de la partie d'introduction d'endoscope, et un surtube (300), dans lequel le surtube (300) est destiné à guider la partie d'introduction d'endoscope (102) et la partie d'introduction d'outil de traitement (202) dans une cavité corporelle, et dans lequel le surtube (300) inclut un corps de surtube (320), lequel passe à travers une paroi corporelle et est introduit dans la cavité corporelle, un passage pour introduction d'endoscope (306), lequel est prévu dans le corps de surtube (320) et permet l'introduction de la partie d'introduction d'endoscope (102) à travers lui de manière à ce que celle-ci puisse se déplacer vers l'avant et l'arrière, et un passage pour introduction d'outil de traitement (308), lequel est prévu dans le corps de surtube (320) et permet l'introduction de la partie d'introduction d'outil de traitement (202) à travers lui de manière à ce que celle-ci puisse se déplacer vers l'avant et l'arrière.

6. Dispositif chirurgical endoscopique (10), selon la revendication 5, comprenant en outre :
un élément de verrouillage réciproque (400) incluant une partie couplée à l'endoscope (420) couplée à la partie d'introduction d'endoscope (102) introduite à travers le passage pour introduction d'endoscope (306) et une partie couplée à l'outil de traitement (422) couplée à la partie d'introduction d'outil de traitement (202) introduite à travers le passage pour introduction d'outil de traitement (308), l'élément de verrouillage réciproque (400) étant agencé dans le corps de surtube (320) de manière à pouvoir être déplacé vers l'avant et vers l'arrière,
dans lequel l'élément de verrouillage réciproque (400) inclut une zone morte où le déplacement vers l'avant et vers l'arrière soit de l'endoscope (100), soit de l'outil de traitement (200) ne se verrouille pas sur le déplacement de l'autre élément, et une zone de détection où le déplacement vers l'avant et vers l'arrière soit de l'endoscope (100), soit de l'outil de traitement (200) se verrouille sur le déplacement de l'autre élément.
